# EUROPEAN PATENT APPLICATION

(11) **EP 1 965 205 A1**
(43) Date of publication of application: **03.09.2008**
(21) Application number: 06834490.2
(22) Date of filing: 12.12.2006
(51) Int. Cl.: G01N 27/62, G01N 27/447, G01N 33/50, G01N 33/68

(54) **AMINO ACID ANALYSIS METHOD USING MASS SPECTROMETER**

(30) Priority: 16.12.2005 JP 2005363512
(71) Applicant: Ajinomoto Co., Inc., Tokyo 104-8315 (JP); Shimadzu Corporation, Kyoto-shi, Kyoto 604-8511 (JP)
(72) Inventor: YAMADA, Naoyuki, Kawasaki.shi Kanagawa 210-8681 (JP); AKASHI, Satoko, Yokohama-shi Kanagawa 221-0005 (JP); SUZUKI, Koichi, Kyoto-shi Kyoto 604-8511 (JP)
(74) Representative: Nicholls, Kathryn Margaret
(86) International application number: PCT/JP2006/324735
(87) International publication number: WO 2007/069591

(57) **Abstract**

A pretreatment method of samples, in which injections of samples are performed efficiently and precisely when amino acids are analyzed with a mass spectrometer, is provided. For the analysis method of samples including analyte comprising amino acid, amine and/or peptide with mass spectrometry, said analyte is derivatized with a modification reagent, said derivative is subjected to a microchip electrophoresis, and then eluate from said microchip electrophoresis is introduced into a mass spectrometer.

## Description

### TECHNICAL FIELD

The present invention relates to an analysis method of amino acid and the like using a mass spectrometer, especially a method for performing a mass spectrometry of amino acid and the like efficiently by a microchip electrophoresis due to a pretreatment of samples to be analyzed and a supplying method of samples for the above method of the mass spectrometry.

### BACKGROUND ART

For a method for analyzing amino acid, a method using an amino acid analyzer is the most precise and it has been popularized widely. However, there are problems of very long analysis time of 1 to 2 hours and relatively low sensitivity of 10 to 50 pmol. In order to overcome the problem of the sensitivity, a method for performing ultraviolet labeling or fluorescence labeling has been developed and its sensitivity has been improved to around 100 fmol by fluorescence detection, but a further improvement of the sensitivity has been expected. In addition, the problem of the analysis time has not been solved yet. Recently, a shortening of the analysis time has been achieved together with an improvement of the sensitivity by combining the fluorescent labeling with a liquid chromatography mass spectrometer (herein below, it is called as LC-MS) (See Patent Document 1). By using this method, the analysis time can be cut greatly as 20 minutes. The sensitivity depends on a performance of mass spectrometers, but it can be quantified at most several fmol if using an expensive tandem mass spectrometer.

However, demands for analyzing amino acid are widely, and a further sensitivity and a high speed of the analysis are expected. As far as the liquid chromatography is used, improvement of performance has reached to its limit and a development of new method not using the liquid chromatography is expected.

On the other hand, capillary electrophoresis is used as a separation method of very small amount of charged substance like ion, organic acid, amino acid, peptide, protein, nucleic acid, saccharide and so on. The capillary electrophoresis is a general method to separate a charged molecule in a solution. A method for analyzing amino acid by CE/MS/MS combining the capillary electrophoresis (CE) with tandem mass spectrometry (MS/MS) is known (See Non-Patent Document 1). The analysis time is 15 minutes and the sensitivity is several fmol even though using this method, so a great improvement has not been achieved yet. On the other hand, a micro total analysis system (*µ* -TAS) which accumulated miniaturized conventional analysis instruments and reaction instruments on a chip substrate has been researched and developed vigorously in recent years and it has reached to a practical use level. A method of performing the capillary electrophoresis (microchip electrophoresis : µ chip CE) by using a microchip provided with fine processing on a base material such as glass substrate and polymers is a main technique of the µ -TAS (See Non-Patent Documents 2 and 3). Also, µ chip CE/MS, in which a mass spectrometer as a detector is connected to the µ chip CE, is a superior instrument which is very sensitive and able to obtain information of mass. By using the µ chip CE/MS or the capillary electrophoresis-MS, amino acids and peptides and the like can be separated and analyzed around 90 seconds to 15 minutes (See Patent Document 2 and Non-Patent Document 4).

Sample migration and injection in the µ chip are performed using potential difference. A method is used in which plural reservoirs including sample, buffer and reagent are connected in fine channels and charged molecule like sample are migrated due to voltage difference between reservoirs. In order to perform the separation and quantification analysis precisely using the µ chip CE, it is important to control the injection volume of sample accurately. In order to inject sample more accurately, a microchip having a structure for regulating sample volume has been developed (See Patent Documents 3, 5 and 6).

On the other hand, a spray ionization mass spectrometer is a high-throughput analysis instrument which can measure mass in high sensitivity and within a several minutes. A bottleneck for short time analysis in the mass spectrometer is injection time of sample. Especially, a required time for introducing samples takes at least 1 minute or more when continuous analysis is performed with an existent auto injector, thereby it cannot make use of performance of the mass spectrometer enough. As a method for supplying samples to the mass spectrometer faster, there is a system using an acoustic injector (See Patent Document 4). This method is as follows. That is, using a microwell plate containing solution sample of multiple specimen, droplets are generated by acoustic pulses successively from samples in the microwell plate to be supplied to the mass spectrometer. However, this method has not been realized yet. Moreover, it is impossible in principle to combine this method with the µ -TAS which is expected to be developed in the future.

[Patent Document 1] WO03/069328
[Patent Document 2] Japanese Patent Kokai Publication No. JP-P2001-83119A
[Patent Document 3] Japanese Patent Kohyo Publication No. JP-A-10-507516
[Patent Document 4] Japanese Patent Kokai Publication No. JP-P2004-205510A
[Patent Document 5] Japanese Patent Kokai Publication No. JP-P2005-164242A
[Patent Document 6] Japanese Patent Kokai Publication No. JP-P2001-24213 7A
[Non-Patent Document 1] Soga et al., Electrophoresis. 2004 Ju1;25 (13):1964-72
[Non-Patent Document 2] Gerard J. M. Bruin, Electrophoresis 2000,21,3931-3951
[Non-Patent Document 3] Lee,S.J. and Lee,S.Y.,Appl.Microbiol.Biotechno1.,2004,64,289-299
[Non-Patent Document 4] Y.Tachibana, K.Otsuka, S.Terabe, A. Arai, K. Suzuki, S. Nakamura, J. Chromatogra. A, 2004, 1025, 287-296
[Non-Patent Document 5] The Japanese Biochemical Society, New Biochemical Experiment Course 1, Protein IV structural activity correlation, Chapter 2
[Non-Patent Document 6] The Japanese Biochemical Society, Forth version Experimental Chemistry Course 22, Organic Synthesis IV, Acid/Amino Acid/Peptide, Chapter 2 third section, Synthesis of protective amino acid, Maruzen

### DISCLOSURE OF THE INVENTION

### PROBLEMS TO BE SOLVED BY THE INVENTION

In an analysis method using a microchip, it has been made an effort to adjust injection volume of samples precisely. On the other hand, the capillary electrophoresis is a technique to separate depending on differences of electric properties of object materials to be measured. Therefore, in the case of introducing samples into separation channels in a microchip electrophoresis, each mobility of samples is different depending on differences of electric properties of object materials to be measured. In the case of mixture samples comprising plural compounds, because each mobility of mixture samples to introduce into the separation channels is different even if injection volume of samples can be uniform, there is liability to change the existence ratio of compounds in the whole sample solution. This phenomenon is serious problem for performing a quantitative analysis with the µ chip CE, and this phenomenon causes a decrease of the signal intensity of the detection peak. Especially, in the case of compounds in which electric properties are greatly different like amino acids, saccharide, peptides and organic acids, it is more serious problem because the signal intensity of the detection peak greatly depends on pH and salt concentration of buffer to be used.

In conventional -technique having such kinds of problems, the present invention resides in providing a pretreatment method of samples, in which injections of samples are performed efficiently and precisely when amino acids are analyzed with a mass spectrometer.

### MEANS TO SOLVE THE PROBLEMS

The present inventors have made investigations to solve the above problems zealously. Namely, a derivatization of amino acids is performed with a modification reagent, the amino acid derivatives are subjected to a microchip electrophoresis, and then the amino acid derivatives are introduced into a mass spectrometer. Thereby, they have found that not only the injections of the samples are performed efficiently but also the precision of the injection volume is improved. Thus, the present inventors have finally completed this invention. That is, the present invention includes the following contents.

(1) An analysis method of samples including analyte comprising amino acid(s), amine(s), and/or peptide(s) in a mass spectrometry, in which the analyte is derivatized with a modification reagent and the derivative is subjected to a microchip electrophoresis and then eluate from the microchip electrophoresis is introduced into a mass spectrometer.

(2) The analysis method according to the above (1), wherein the derivatization process is that one of amino group and imino group of the analyte is converted into any one of carbamoyl group, thiocarbamoyl group, tertiary amine and quaternary ammonium salt.

(3) The analysis method according to the above (1), wherein the derivative has a structure of tertiary amine or quaternary ammonium salt having aromatic ring and the structure is easy to ionize in the mass spectrometry.

(4) The analysis method according to any one of the above (1) to
(3), in which the derivative has a structure shown in any one of following general formulae (1) to (9),

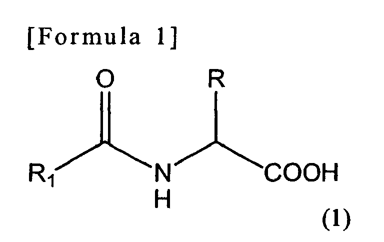

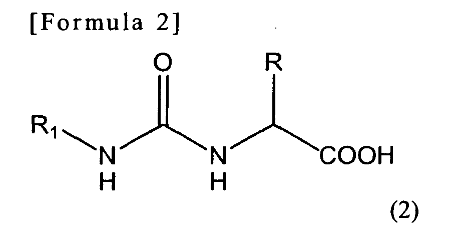

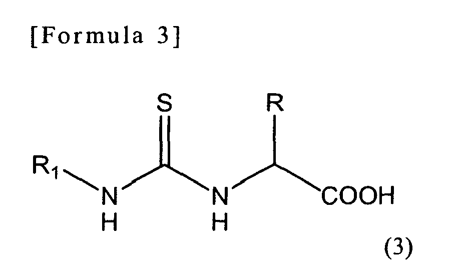

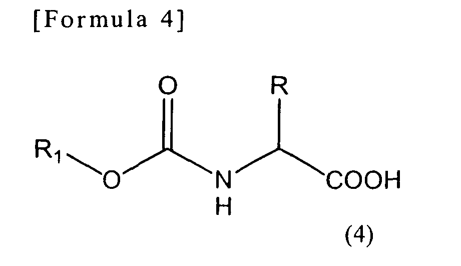

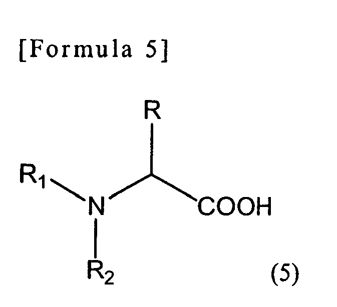

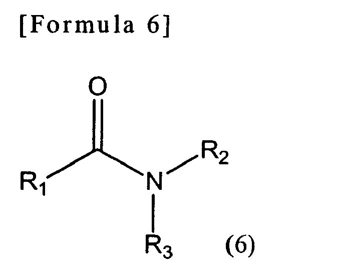

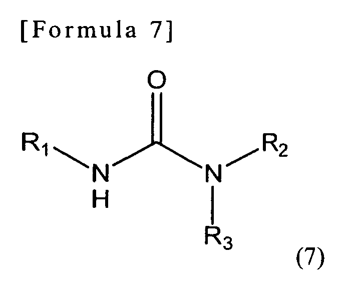

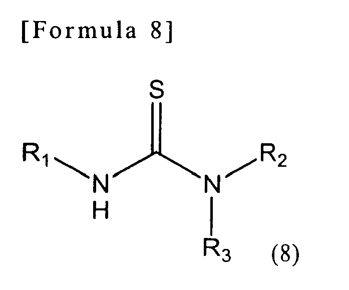

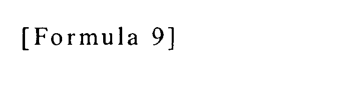

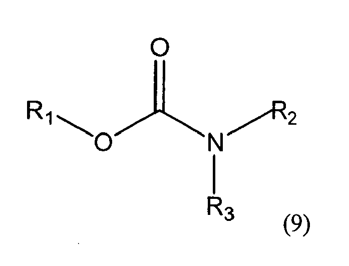

wherein in the above formulae (1) to (9), R represents a hydrogen atom or an alkyl group which may have a substituent group and is a side chain of an amino acid, R₁ represents alkyl group which may have a substituted group or a substituted or unsubstituted group having aromatic carbocyclic ring or aromatic heterocyclic ring, R₂ and R₃ represent alkyl group which may have a substituted group independent mutually, R₂ and R₃ may form a ring together, or when one of R₂ and R₃ represents amino acid residue of peptide, the other can be hydrogen atom.

(5) The analysis method according to any one of the above (1) to (4), wherein the modification reagent is a compound selected from the group consisting of acetic aid anhydride, N-acetyl-imidazole, N-acetyl-succinimide, N-acetyl-imidoacetate, N-acetyl-imidazole, Bolton-Hunter reagent, carbamate compound, isothiocyanate compound, N-hydroxy-succinimide-ester, dansyl-chloride, dabsyl-chloride, and dansyl-fluoride and NBD-F(4-Fluoro-7-nitrobenzofurazan).

(6) The analysis method according to the above (5), wherein the carbamate compound is selected from the group consisting of 6-aminoquinolyl-N-hydroxysuccinimidyl-carbamate(AQC), p-dimethylaminoanilyl-N-hydroxysuccinimidyl-carbamate(DAHS), 3-aminopyridyl-N-hydroxysuccinimidyl-carbamate(APDS), p-trimethylammoniumanilyl-N-hydroxysuccinimidyl-carbamate-iodide( TAHS), aminopyrazyl-N-hydroxysuccinimidyl-carbamate, 9-aminoacridyl-N-hydroxysuccinimidyl-carbamate and 1-naphthylamino-N-hydroxysuccinimidyl-carbamate.

(7) The analysis method according to the above (5), wherein the isothiocyanate compound is phenyl isothiocyanate or fluorescein isothiocyanate.

(8) The analysis method according to any one of the above (1) to (7), wherein the mass spectrometer is one of an electro-spray-ionization mass spectrometer, an atmospheric pressure chemical ionization mass spectrometer, a cold-spray-ionization mass spectrometer and a laser-spray-ionization mass spectrometer.

(9) A method for supplying samples including plural analytes comprising amino acid(s), amine(s) and/or peptide(s) to an analysis instrument, in which the analytes are reacted with a modification reagent to prepare any one of derivatives shown in the above general formulae (1) to (9), then an electrophoresis of the derivative is performed with a microchip electrophoresis device, and then eluate from the microchip electrophoresis is supplied to an inlet(s) of the analysis instrument.

(10) A pretreatment instruments for analyzing samples including plural analytes comprising amino acid(s), amine(s) and/or peptide(s) with a mass spectrometer, in which the pretreatment instrument has a reaction part preparing the derivative described in the above (9) by reacting the analytes with a modification reagent and a microchip electrophoresis part performing an electrophoresis of the derivative.

### MERITORIOUS EFFECTS OF THE INVENTION

According to the present invention, each of sample introduction for analyzing amino acid by the mass spectrometer is performed efficiently, thereby many samples can be analyzed in a short time compared to the conventional method. Also, the precision of the injection is improved and the quantifiability is improved, too.

### BRIEF DESCRIPTION OF THE DRAWINGS

Figure 1A : Mass elecropherogram of analyzing 17 amino acids in Example 1 ;
Figure 1B : Mass elecropherogram of analyzing 17 amino acids in Example 1 ;
Figure 2 : Mass elecropherogram (left) and mass specta (right) of analyzing 17 amino acids in Example 2; and
Figure 3 : Mass elecropherogram of analyzing a mixture of 4 amino acids in Example 3.

### PREFERRED MODES FOR CARRYING OUT THE INVENTION

When samples are migrated by potential difference in the µ -TAS, each mobility of samples is different depending on differences of electric properties of object materials to measure. In the case of mixture samples comprising plural compounds, even if injection volume of samples can be made uniform, there is liability to cause a change in the existence ratio of compounds in the sample solution because each mobility of compounds mixture samples to reach the injection part is different. This phenomenon is serious problem especially for performing a quantitative analysis with the µ -TAS, and this phenomenon causes a decrease in the signal intensity of the detection peak and a deterioration of quantitative sensitivity. Especially, in the case of compounds whose electric properties are greatly different like amino acids, saccharide, peptides and organic acids, it is more serious problem because the signal intensity of the detection peak greatly depends on pH and salt concentration of buffer to be used pKa of biologic molecules like amino acids, peptides, organic acids and nucleic acids have variety around neutral neighborhood. This variety of pKa is expressed as a difference of mobility. This is remarkable, especially for amino acids having amino group and carboxyl group. The pKa of amino group is greatly different depending on kind of amino acids. Therefore, in the method of the present invention, amino group is modified with a modification reagent not to have basicity or introduction of molecules having larger pKa or smaller pKa, so it is possible to reduce the difference of pKa for the method of the present invention. Thereby, the difference of mobility when introducing samples can be reduced.

For the present invention, samples which become the object of the analysis include analyte comprising amino acids, amine (primary amine, secondary amine and the like) and/or peptides. These analytes are compounds (they may be in the form of salt) having amino group(s) and/or imino group(s) in molecule, and amino group and imino group may be one or plural. Also, analytes existing in samples may be one kind or mixture of plural kinds, but the present invention takes effect in the case of analytes including plural compounds. In concrete, analytes include 20 kinds of natural amino acids, in addition hydroxylysine and hydroxyproline or non-natural amino acids such as homocysteine and homoserine and the like, and amines such as histamine and ornithine and the like. Analytes may include a plurality of kinds of such compounds. Peptides, in which several amino acids are connected to form dipeptide or tripeptide, are also encompassed in the analytes of the present invention. In recent years, proteomics aimed for comprehensive analysis of protein has been played an important role in the life science research field. In general proteomics, object protein to be analyzed is digested by trypsin to make peptide fragments and measured with the mass spectrometer. Because trypsin is an enzyme to digest protein at carboxyl terminus of lysine residue or arginine residue, peptides to be generated are peptides having one residue of lysine or arginine at C terminal. Since peptides prepared in such way have limited reaction sites with the modification reagent concerning the present invention, they can be analyzed easily by the method of the present invention as well as amino acid or amine.

Many means are known for a derivatization method of amino group of amino acids (See e.g., Non-Patent Documents 5). As a derivatization method in which positive charge of amino group is maintained, there are derivatizations of guanidine or amidine. For regulation of pKa which is main point of the present invention, it is preferred to convert amino group into carbamoyl group by carbamoyl derivatization or acetylation, or into thiocarbamoyl group by thiocarbamoyl derivatization. As the acetylation reagent, there are acetic aid anhydride, N-acetyl-imidazole, N-acetyl-succinimide, N-acetyl-imidoacetate, N-acetyl-imidazole, Bolton-Hunter reagent and the like. Also, carbamate compound, as is well known for labeling amino group of amino acids or peptides, isothiocyanate compound, N-hydroxy-succinimide-ester, and alkylating agent(s) like dansyl-chloride, dabsyl-chloride, dansyl-fluoride and the like can be used. In the concrete, carbamate compound to generate derivatives described in the above formula (1) by reacting with amino acids is preferred. In more detail example, 6-aminoquinolyl-N-hydroxysuccinimidyl-carbamate(AQC), p-dimethylaminoanilyl-N-hydroxysuccinimidyl-carbamate(DAHS), 3-aminopyridyl-N-hydroxysuccinimidyl-carbamate(APDS), p-trimethylammoniumanilyl-N-hydroxysuccinimidyl-carbamate-iodide( TAHS), aminopyrazyl-N-hydro xysuccinimidyl-carbamat e, 9-aminoacridyl-N-hydroxysuccinimidyl-carbamate, 1-naphthylamino-N-hydroxysuccinimidyl-carbamate and the like are preferred. Also, isothiocyanate compound(s) to generate derivatives described in the above formula (2) by reacting with amino acid(s) is listed, in more detail, phenyl isothiocyanate, fluorescein isothiocyanate and the like are listed. In addition, amino group can be converted into carbamoyl group by introducing general protective group of amino group such as benzyloxycarbonyl(Z) group, t-butoxycarbonyl(Boc) group or 9-fluorenylmethoxycarbonyl(Fmoc) group (See e.g., Non-Patent Document 6).

Also, in order to improve the sensitivity of the mass spectrometry, a derivatization having charge is more preferred. Considering the above charge regulation effect, derivatives having tertiary amine or quaternary ammonium salt having aromatic ring are more preferred. In more detail example, 6-aminoquinolyl-N-hydroxysuccinimidyl-carbamate(AQC), p-dimethylaminoanilyl-N-hydroxysuccinimidyl-carbamate(DAHS), 3-aminopyridyl-N-hydroxysuccinimidyl-carbamate(APDS), p-trimethylammoniumanilyl-N-hydroxysuccinimidyl-carbamate-iodide( TAHS), aminopyrazyl-N-hydroxysuccinimidyl-carbamate, 9-aminoacridyl-N-hydroxysuccinimidyl-carbamate or 1-naphthylamino-N-hydroxysuccinimidyl-carbamate and the like can be used, and an effect for improving the sensitivity in the mass spectrometry is also achieved.

Derivatized amine or amino acid can be detected and quantified by performing the microchip electrophoresis and analyzing the mass spectrometer. Since a mass separation can be performed with the mass spectrometer without performing separation of compounds in a microchip, channels length of a microchip usually used for separation can be shortened as much as possible, then great cut of an analysis time can be realized. Thereby, an auto-injector which can inject accurate volume is made without changing the ratio of sample composition or concentration of sample. As a result, according to the present invention, the stabilization of the quantity of introduction samples, the high sensitivity and the high speed of the analysis time can be achieved at the same time.

On the other hand, in the microchip electrophoresis, there is a method to use reverse-phased carrier, besides the capillary electrophoresis. By performing together with this method, compounds having same mass can be separated, for example in amino acids, leucine and isoleucine can be separated.

In general, for the *µ* -TAS, a potential difference is frequently used when samples or reagent are migrated. Therefore, according to the present invention, it can be possible to have uniform mobility for compounds having different mobilities, and it can be widely applied to the µ-TAS.

As a mass spectrometry used in the present invention, a method is used wherein liquid containing samples eluted from the above microchip electrophoresis is sprayed into mist, followed by introduction into a spraying instrument for ionization, and then the sample is measured in a gas phase. As the spraying instrument, there are an electro-spray-ionization method (ESI), an atmospheric pressure chemical ionization method (APCI), a cold-spray-ionization mass spectrometer (CSI), a laser-spray-ionization method (LSI) and the like, but it is not limited to the above listed. Generated ions are applied to the mass spectrometry, and they are separated into with mass-to-charge ratio (*m*/*z*) by applying various different voltages to electrode. This mass analysis part plays an important role for sensitivity and resolution of analyzed data, accuracy of mass, or abundant information obtained from mass spectrum data. The separation methods of ions, may be currently classified into six basic types, that is, magnetic field type, electric field type, ion-trap type, time-of-flight (TOF) type, quadrupole type and Fourier transform cyclotron type. They have positive aspect and negative aspect, respectively, and they can be used in single or in combination each other, whereas a quadrupole mass spectrometer is usually used for ionization due to the ESI. In addition, it provides certainty in the measurement and interpretation of multiply-charged ions by connecting plural quadrupoles in tandem (MS/MS).

In below, the present invention will be described in further detail by the way of examples, but the present invention is not limited by these examples.

### EXAMPLE 1

### Derivatization of amino acids

20 µ l of 17 kinds of amino acids mixture standard solution, Type H (Wako Jyunyaku) was added to 60 µ l of boric acid buffer (0.2M borate, pH8.8), and mixed well. Then, 20 µ l of 6-aminoquinolyl-N-hydroxysuccinimidyl-carbamate(AQC) standard reagent solution (3-5mg of AQC was dissolved in 1 ml of acetonitrile or reagent powder contained in AccQ-Fluor(Trademark) Reagent Kit by Nihon Waters was dissolved in 1ml of reagent diluting solution) was added to this mixture. The obtained mixture was heated at 55 degree Celsius for 10 minutes. The derivatized amino acid mixture was diluted in 10mM (NH₄)₂CO₃ dilution buffer (pH 8.7), and it was measured in a *µ* chip electrophoresis mass spectrometer.

### Measurement of amino acid with modified amino group by the µ chip electrophoresis mass spectrometer

The µ chip electrophoresis mass spectrometer was used by connecting a µ chip electrophoresis instrument (this is the same instrument as disclosed in Patent Document 2 and Non-Patent Document 4) equipped with an ESI emitter to a commercially available mass spectrometer.

### Conditions for µ chip electrophoresis

Material of microchip was quartz and channel shape was as follows: width of the channel was 82 µ m; depth of the channel was 36 µ m; and length of separation channel was 59 mm. As a treatment for channel surface, Positive EOF (silanol activation by alkaline) or Negative EOF (coated with PolyE-323) was used. As the ESI emitter, Picotip (FS360-50-15-N, New Objective) was used.

### Conditions for electrophoresis

Sample introduction: Gate Injection method
Potential gradient: +400V/cm (Positive EOF)
   -400V/cm (Negative EOF)
Gate ratio: 2.0
ESI voltage: 3.0kV

### Measurement conditions for the mass spectrometer

Instruments for measurement: ESI-Q-tof-2 (Micromass)
Measuring range for mass: m/z 160-800
Scan time: 1 second (1 scan is integration for 1 second)
Time between scans: 0.1 second
Cone voltage: 30V
Collision voltage: 10V
Data processing: MassLynx v.3.5(Micromass)

### Result

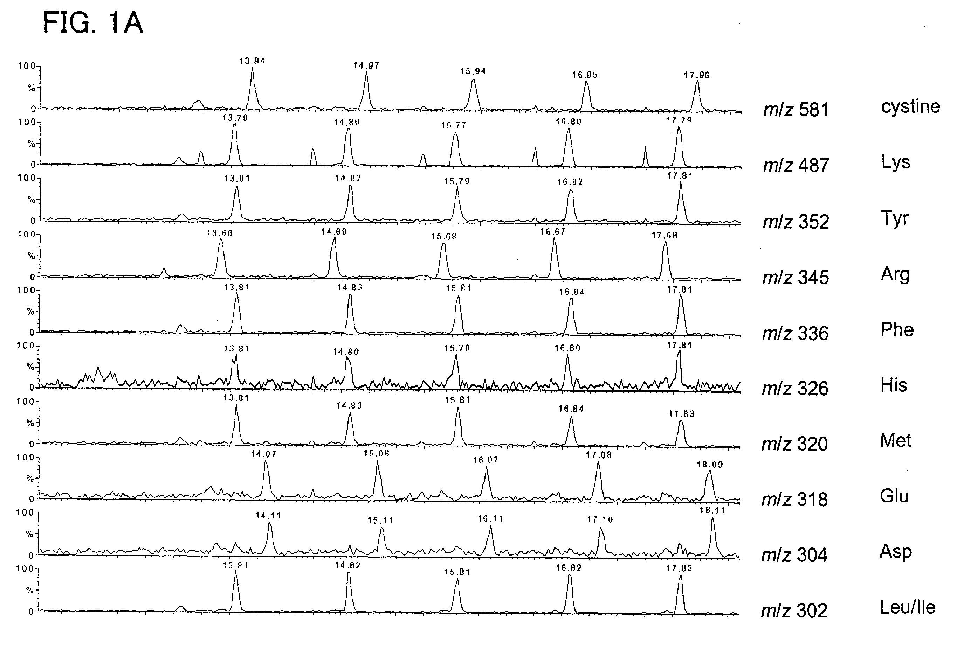

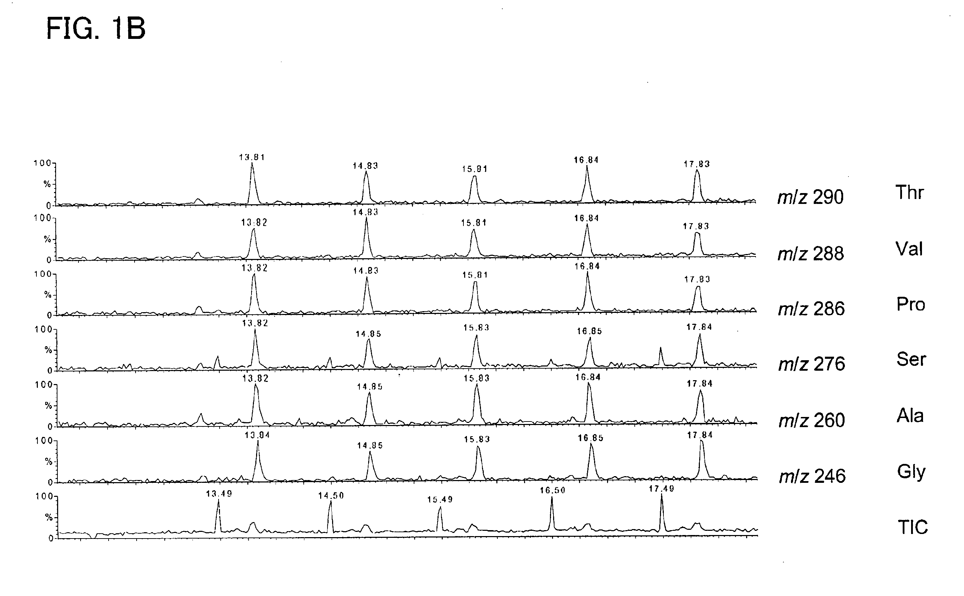

Mass electropherogram for analyzing samples of 17 kinds amino acids derivatized with AQC at the same time by using non-coating microchip are shown in Figures 1A and 1B. Samples were introduced for 1 second with the Gate Injection method at interval of 1 minute. All 17 kinds of amino acids derivatized with AQC were detected in every 1 minute.

Reproducibility of the samples introduction interval at this time is shown in following Table 1. Reproducibility of 5 times measurement was very accurate for all amino acids.

**[Table 1]**

| # | 1 | 2 | 3 | 4 | 5 | average | SD | RSD(%) |
|---|---|---|---|---|---|---|---|---|
| Gly | 59.4 | 60.0 | 58.8 | 60.0 | 59.4 | 59.52 | 0.502 | 0.8 |
| Ala | 58.2 | 60.0 | 58.8 | 59.4 | 59.4 | 59.16 | 0.684 | 1.2 |
| Ser | 58.2 | 60.0 | 58.8 | 60.0 | 59.4 | 59.28 | 0.782 | 1.3 |
| Pro | 58.2 | 58.8 | 57.6 | 59.4 | 58.8 | 58.56 | 0.684 | 1.2 |
| Val | 58.2 | 58.8 | 57.6 | 59.4 | 58.8 | 58.56 | 0.684 | 1.2 |
| Thr | 57.6 | 58.8 | 57.6 | 59.4 | 58.8 | 58.44 | 0.805 | 1.4 |
| Le/II | 57.6 | 58.2 | 57.6 | 58.2 | 58.8 | 58.08 | 0.502 | 0.9 |
| Asp | 75.6 | 75.6 | 75.6 | 75.0 | 75.6 | 75.48 | 0.268 | 0.4 |
| Glu | 73.2 | 73.8 | 73.2 | 73.8 | 74.4 | 73.68 | 0.502 | 0.7 |
| Met | 57.6 | 58.8 | 57.6 | 59.4 | 58.8 | 58.44 | 0.805 | 1.4 |
| His | 57.6 | 57.0 | 56.4 | 57.0 | 57.6 | 57.12 | 0.502 | 0.9 |
| Phe | 57.6 | 58.8 | 57.6 | 59.4 | 57.6 | 58.20 | 0.849 | 1.5 |
| Arg | 48.6 | 49.8 | 49.8 | 49.2 | 49.8 | 49.44 | 0.537 | 1.1 |
| Tyr | 57.6 | 58.2 | 56.4 | 58.2 | 57.6 | 57.60 | 0.735 | 1.3 |
| Lys | 56.4 | 57.0 | 55.2 | 57.0 | 56.4 | 56.40 | 0.735 | 1.3 |
| cystine | 65.4 | 67.2 | 65.4 | 66.0 | 66.6 | 66.12 | 0.782 | 1.2 |

In the same way, a peak area, that is, reproducibility of quantifiability is shown in Table 2. Very high reproducibility was indicated for all amino acids when measuring 5 times.

**[Table 2]**

| # | 1 | 2 | 3 | 4 | 5 | average | SD | RSD(%) |
|---|---|---|---|---|---|---|---|---|
| Gly | 2.491 | 2.138 | 2.510 | 2.433 | 2.957 | 2.506 | 0.293 | 11.7 |
| Ala | 3.358 | 2.566 | 3.241 | 2.866 | 2.613 | 2.929 | 0.360 | 12.3 |
| Ser | 3.225 | 2.969 | 2.923 | 2.828 | 3.078 | 3.005 | 0.153 | 5.1 |
| Pro | 3.901 | 3.163 | 3.042 | 3.354 | 3.054 | 3.303 | 0.357 | 10.8 |
| Val | 5.148 | 5.436 | 4.916 | 4.785 | 4.376 | 4.932 | 0.397 | 8.1 |
| Thr | 3.976 | 3.888 | 3.334 | 3.558 | 3.673 | 3.686 | 0.258 | 7.0 |
| Le/I1 | 11.206 | 10.707 | 10.535 | 11.236 | 11.18 | 10.973 | 0.327 | 3.0 |
| Asp | 2.748 | 2.365 | 2.343 | 2.298 | 2.783 | 2.507 | 0.237 | 9.5 |
| Glu | 3.440 | 2.943 | 2.550 | 3.102 | 3.054 | 3.018 | 0.321 | 10.6 |
| Met | 5.636 | 5.724 | 6.701 | 5.477 | 5.154 | 5.738 | 0.580 | 10.1 |
| His | 1.162 | 1.386 | 1.684 | 1.122 | 1.436 | 1.358 | 0.228 | 16.8 |
| Phe | 8.701 | 7.791 | 8.605 | 8.566 | 8.678 | 8.468 | 0.382 | 4.5 |
| Arg | 9.173 | 8.932 | 8.362 | 8.241 | 7.805 | 8.503 | 0.550 | 6.5 |
| Tyr | 9.187 | 8.319 | 8.424 | 8.611 | 8.461 | 8.600 | 0.344 | 4.0 |
| Lys | 16.872 | 16.174 | 15.056 | 17.615 | 16.345 | 16.412 | 0.943 | 5.7 |
| cystine | 5.722 | 5.101 | 6.019 | 4.859 | 4.877 | 5.316 | 0.526 | 9.9 |

### EXAMPLE 2

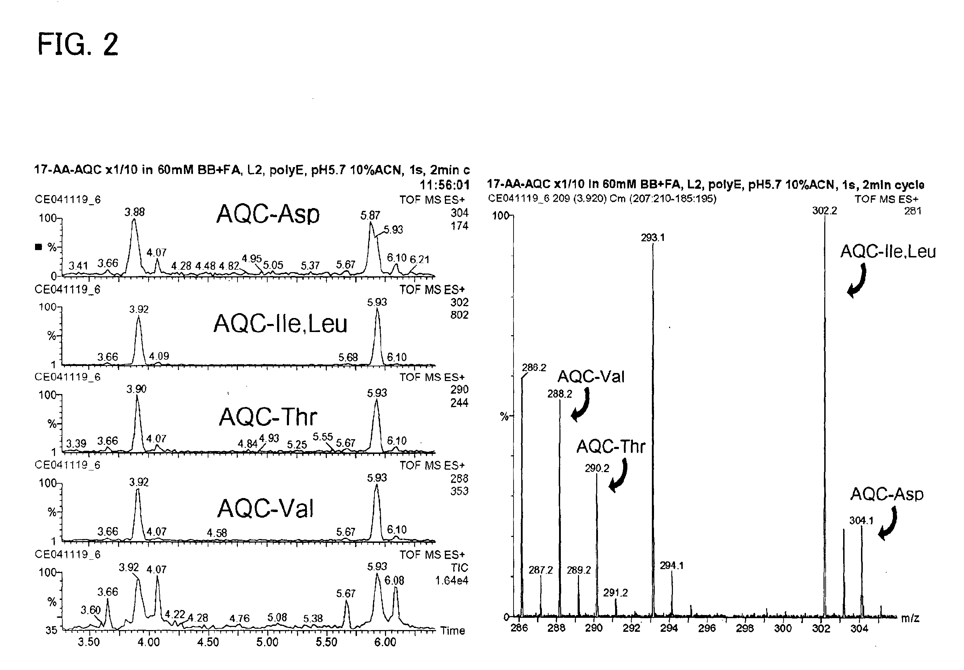

Mass electropherogram and mass spectrum resulted from performing mass spectrometry which implements 1 second introduction in every 2 minutes for samples of 17 kinds amino acids derivatized with AQC at the same time as well as same method in Example 1 by using PolyE-323 coating microchip are shown in Figure2. Amino acids derivatized with AQC could be detected accurately at intervals of 2 minutes.

### EXAMPLE 3

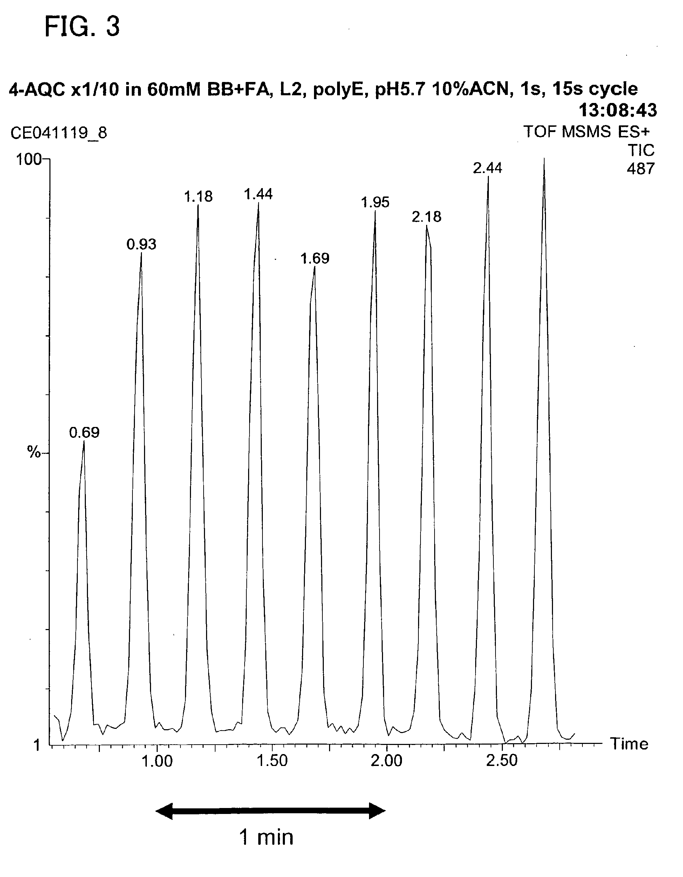

Mass electropherogram resulted from performing mass spectrometry which implements 1 second introduction with every 15 minutes interval for samples of amino acid mixture made with 4 kinds of Leu, Glu, Phe and Arg derivatized with AQC as well as same method in Example 1 by using PolyE-323 coating microchip is shown in Figure 3. Samples could be introduced correctly even at every 15 seconds interval and mass of samples could be measured. In this example, samples introduction was performed at every 15 seconds interval, but it is possible to perform at an interval of every 2 to 3 seconds.

## Claims

1. An analysis method of samples including analyte comprising amino acid(s), amine(s), and/or peptide(s) in a mass spectrometry, in which said analyte is derivatized by a modification reagent and said derivative is subjected to a microchip electrophoresis and then eluate from said microchip electrophoresis is introduced into a mass spectrometer.

2. The analysis method according to claim 1, wherein said derivatization process is that one of amino group and imino group of said analyte is converted into any one of carbamoyl group, thiocarbamoyl group, tertiary amine and quaternary ammonium salt.

3. The analysis method according to claim 1, wherein said derivative has a structure of tertiary amine or quaternary ammonium salt having aromatic ring and said structure is easy to ionize in said mass spectrometry.

4. The analysis method according to any one of claims 1 to 3, in which said derivative has a structure shown in any one of following general formulae (1) to (9), 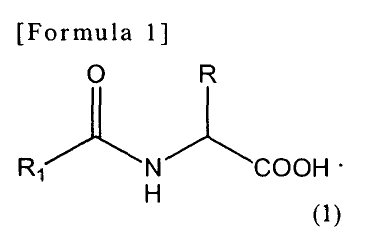 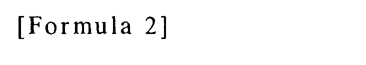 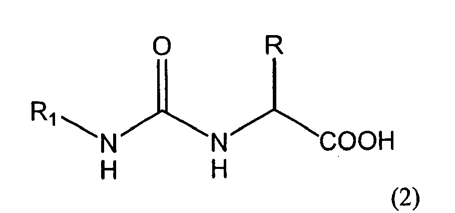 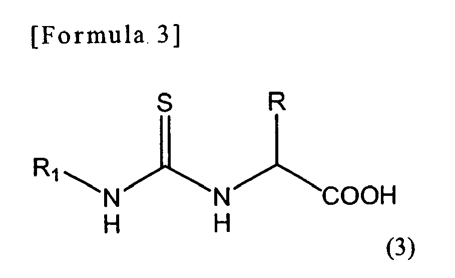 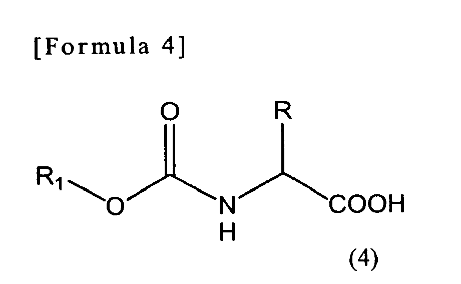 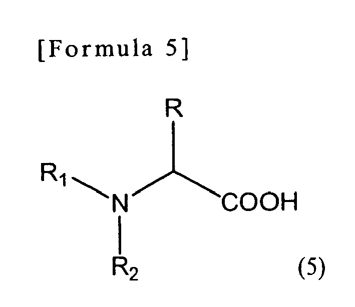 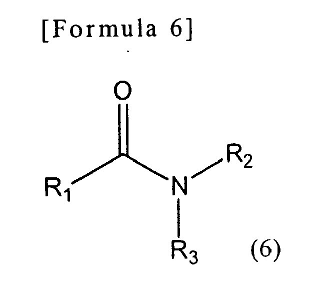 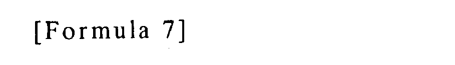 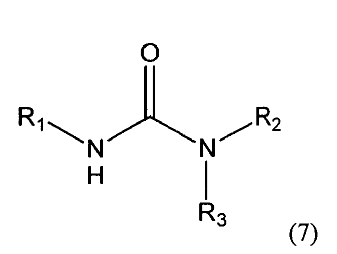 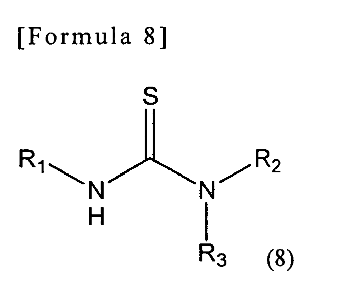 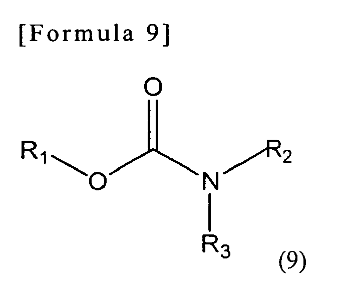 wherein in the above formulae (1) to (9), R represents a hydrogen atom or an alkyl group which may have a substituent group and is a side chain of an amino acid, R₁ represents alkyl group which may have a substituted group or a substituted or unsubstituted group having aromatic carbocyclic ring or aromatic heterocyclic ring, R₂ and R₃ represent alkyl group which may have a substituted group independent mutually, R₂ and R₃ may form a ring together, or when one of R₂ and R₃ represents amino acid residue of peptide, the other can be hydrogen atom.

5. The analysis method according to any one of claims 1 to 4, wherein said modification reagent is a compound selected from the group consisting of acetic aid anhydride, N-acetyl-imidazole, N-acetyl-succinimide, N-acetyl-imidoacetate, N-acetyl-imidazole, Bolton-Hunter reagent, carbamate compound, isothiocyanate compound, N-hydroxy-succinimide-ester, dansyl-chloride, dabsyl-chloride, and dansyl-fluoride and NBD-F(4-Fluoro-7-nitrobenzofurazan).

6. The analysis method according to claim 5, wherein said carbamate compound is selected from the group consisting of 6-aminoquinolyl-N-hydroxysuccinimidyl-carbamate(AQC), p-dimethylaminoanilyl-N-hydroxysuccinimidyl-carbamate(DAHS), 3-aminopyridyl-N-hydroxysuccinimidyl-carbamate(APDS), p-trimethylammoniumanilyl-N-hydroxysuccinimidyl-carbamate-iodide( TAHS), aminopyrazyl-N-hydroxysuccinimidyl-carbamate, 9-aminoacridyl-N-hydroxysuccinimidyl-carbamate and 1-naphthylamino-N-hydroxysuccinimidyl-carbamate.

7. The analysis method according to claim 5, wherein said isothiocyanate compound is phenyl isothiocyanate or fluorescein isothiocyanate.

8. The analysis method according to any one of claims 1 to 7, wherein said mass spectrometer is one of an electro-spray-ionization mass spectrometer, an atmospheric pressure chemical ionization mass spectrometer, a cold-spray-ionization mass spectrometer and a laser-spray-ionization mass spectrometer.

9. A method for supplying samples including plural analytes comprising amino acid(s), amine(s) and/or peptide(s) to an analysis instrument, in which said analytes are reacted with a modification reagent to prepare any one of derivatives shown in following general formulae (1) to (9), then electrophoresis of said derivative is performed with a microchip electrophoresis device, and then eluate from said microchip electrophoresis is supplied to an inlet(s) of said analysis instrument. 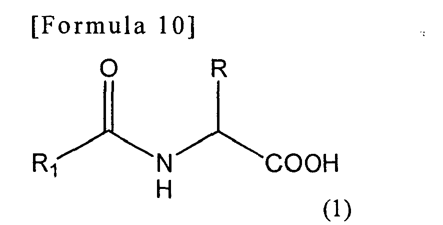 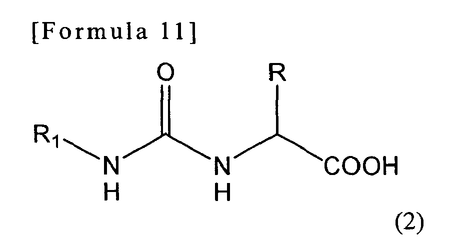 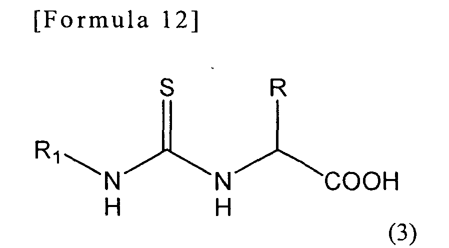 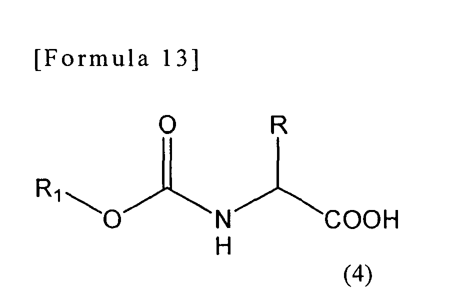 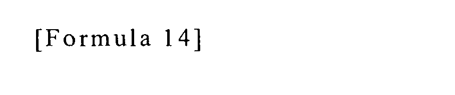 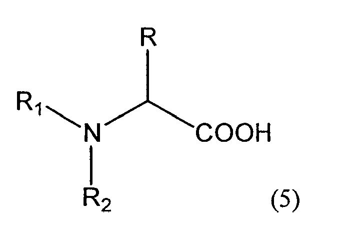 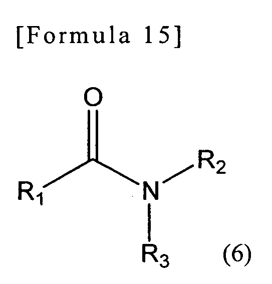 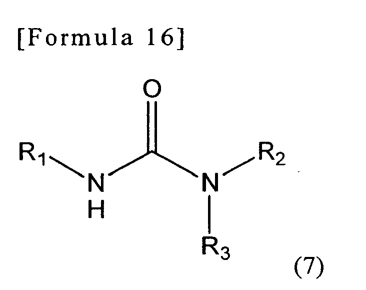 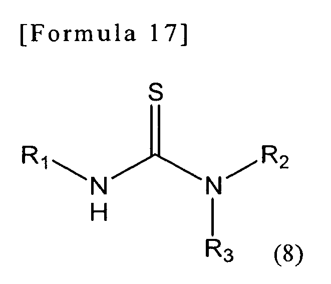 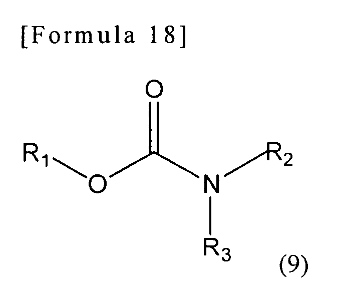 wherein in the above formulae (1) to (9), R represents hydrogen atom which is a side chain of amino acid(s) or alkyl group which may have a substituted group, R₁ represents alkyl group which may have a substituted group or a substituted group including carbocyclic or heterocyclic having aromaticity, R₂ and R₃ represent alkyl group which may have a substituted group independent mutually, R₂ and R₃ may form a ring together, or when one of R₂ and R₃ represents amino acid residue of peptide, the other can be hydrogen atom.

10. A pretreatment instrument for analyzing samples including plural analytes comprising amino acid(s), amine(s) and/or peptide(s) with a mass spectrometer, in which said pretreatment instrument has :
a reaction part preparing the derivative described in claim 9 by reacting said analytes with a modification reagent; and
a microchip electrophoresis part performing an electrophoresis of said derivative.
